# EUROPEAN PATENT SPECIFICATION

(11) **EP 2 272 920 B1**
(45) Date of publication and mention of the grant of the patent: **03.08.2011**
(21) Application number: 10162203.3
(22) Date of filing: 06.05.2010
(51) Int. Cl.: C09B 57/00, H01G 9/20

(54) **Dye for dye-sensitized solar cell and dye-sensitized solar cell including the same**
Farbstoff für farbstoffsensibilisierte Solarzelle und farbstoffsensibilisierte Solarzelle einen derartigen Farbstoff enthaltend
Colorant pour cellule solaire sensibilisée aux colorants et cellule solaire sensibilisée aux colorants l'incluant

(30) Priority: 10.07.2009 KR 20090063233
(43) Date of publication of application: 12.01.2011
(73) Proprietor: Samsung SDI Co., Ltd., Gyeonggi-do (KR)
(72) Inventor: Shin, Byong-Cheol, Kyeonggi-Do (KR); Lee, Ji-Won, Kyeonggi-Do (KR); Kang, Moon-Sung, Gyeonggi-do (KR); Ko, Jae-Jung, Chungcheongnam-do (KR); Choi, Hyun-Bong, Chungcheongnam-do (KR)
(74) Representative: Gulde Hengelhaupt Ziebig & Schneider

(56) References cited:
- JP-A- 2006 265 549
- JP-A- 2008 177 147
- S.ALEX ET AL.: "Dye sensitization of nanocrystalline TiO2: enhanced efficiency of unsymmetrical versus symmetrical Squaraine Dyes" J.PHOTOCHEM.PHOTOBIOL.A, vol. 172, 2005, pages 63-71, XP002588180
- H.QIN ET AL.: "An Organic Sensitizer with a fused Dithienothiophene Unit for efficient an stable Dye-Sensitized Solar Cells" J.AM.CHEM.SOC., vol. 130, 2008, pages 9202-9203, XP002588181
- SAYAMA K ET AL: "Efficient sensitization of nanocrystalline TiO2 films with cyanine and merocyanine organic dyes" SOLAR ENERGY MATERIALS AND SOLAR CELLS, ELSEVIER SCIENCE PUBLISHERS, AMSTERDAM, NL LNKD- DOI:10.1016/S0927-0248(03)00113-2, vol. 80, no. 1, 15 October 2003 (2003-10-15), pages 47-71, XP004456743 ISSN: 0927-0248
- Y.CHEN ET AL.: "Highly efficient co-sensitization of nanocrystalline TiO2 Electrodes with plural Organic Dyes" NEW J.CHEM., vol. 29, 2005, pages 773-776, XP002588182

## Description

The present invention relates to a dye for a dye-sensitized solar cell and a dye-sensitized solar cell including the same.

Attempts have been made to develop energy sources that can replace fossil fuels. Extensive research is underway to find ways for using alternative energy sources, e.g., wind power, atomic power, and solar power, as substitutes for petroleum resources, which may be depleted within several decades. Among the alternative energy sources, solar cells use solar energy that is infinite and environmentally friendly, as opposed to other energy sources. Since 1983 when a Se solar cell was first produced, solar cells have been highlighted, and Si solar cells have recently been drawing attention.

However, it may be difficult to practically use Si solar cells because production costs may be high. Therefore, a dye-sensitized solar cell that may be produced at a low cost has attracted attention.

### SUMMARY

Embodiments are directed to a dye for a dye-sensitized solar cell and a dye-sensitized solar cell including the same, which substantially overcome one or more of the drawbacks, limitations, and/or disadvantages of the related art.

It is a feature of an embodiment to provide a dye for a dye-sensitized solar cell having high efficiency.

It is another feature of an embodiment to provide a dye-sensitized solar cell having improved short-circuit current and photoelectric conversion efficiency and being capable of suppressing dark currents.

At least one of the above and other features and advantages is realized by providing a dye for a dye-sensitized solar cell represented by Formula 1, Formula 2, or a combination thereof: 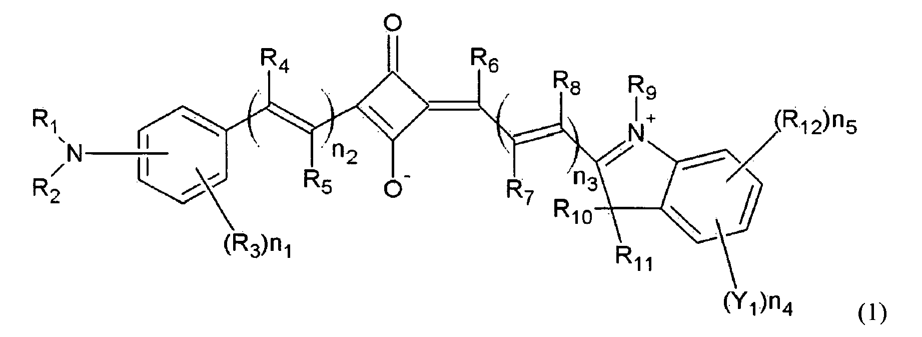 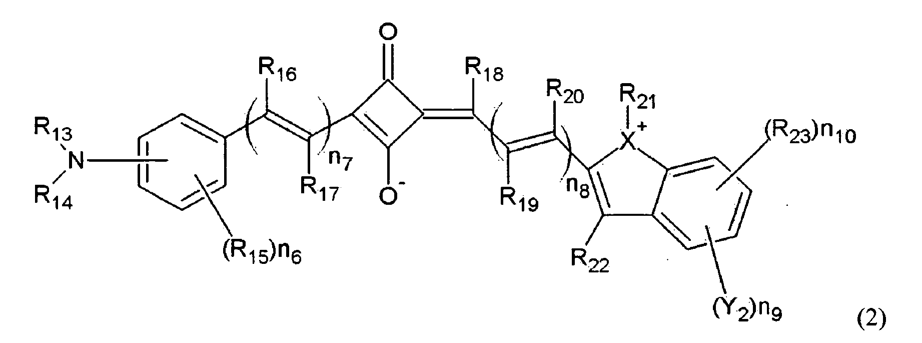 wherein, in Formulae 1 and 2 R₁ to R₃, R₉ to R₁₂, R₁₃ to R₁₅, and R₂₁ to R₂₃ are each independently hydrogen, a substituted or unsubstituted aromatic group, a substituted or unsubstituted heterocyclic group, substituted or unsubstituted aliphatic group, or a substituted or unsubstituted alicyclic group, R₄ to R₈ and R₁₆ to R₂₀ are each independently hydrogen or a substituted or unsubstituted aliphatic group, X is O or S, Y₁ and Y₂ are each independently an acidic functional group or a hydroxy group, n₁ and n₆ are each independently integers of 0 to 4, n₂ and n₇ are each independently integers of 1 to 4, n₃ and n₈ are each independently integers of 0 to 2, n₄ and n₉ are each independently integers of 1 to 4, n₅ and n₁₀ are each independently integers of 0 to 3, n₄+n₅ is an integer of 4 or less, and n₉+n₁₀ is an integer of 4 or less.

R₁, R₂, R₁₃ and R₁₄ may each independently be a substituted or unsubstituted C₉ to C₃₀ aromatic group, a substituted or unsubstituted C₂ to C₃₀ heterocyclic group, a substituted or unsubstituted C₁₃ to C₃₀ aliphatic group, or a substituted or unsubstituted C₃ to C₃₀ alicyclic group.

At least one of R₁, R₂, R₁₃ and R₁₄ may be a substituted or unsubstituted fluorenyl group.

R₉ and R₂₁ may each independently be a substituted or unsubstituted C₅ to C₁₅ aliphatic group.

Y₁ and Y₂ may each independently be a carboxyl group, a sulfonic acid group, a phosphoric acid group, or a hydroxy group.

The dye may be represented by Formula 3-1:

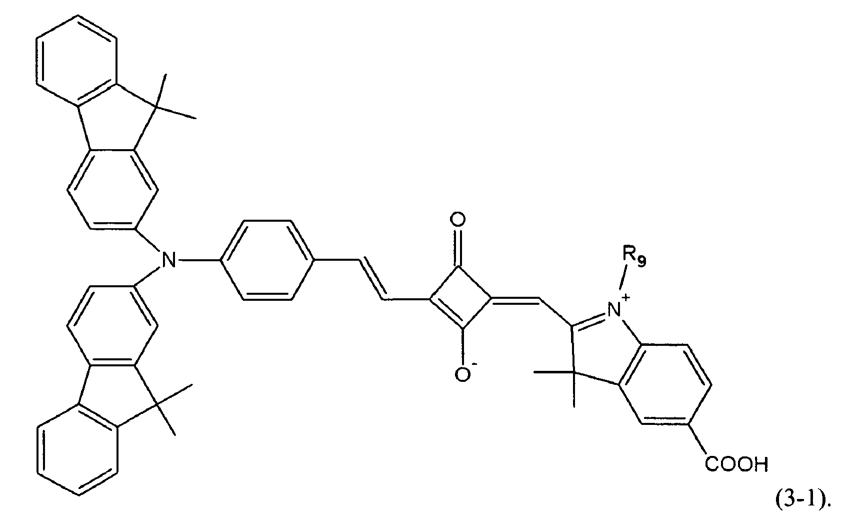

The dye may be represented by Formula 3-2:

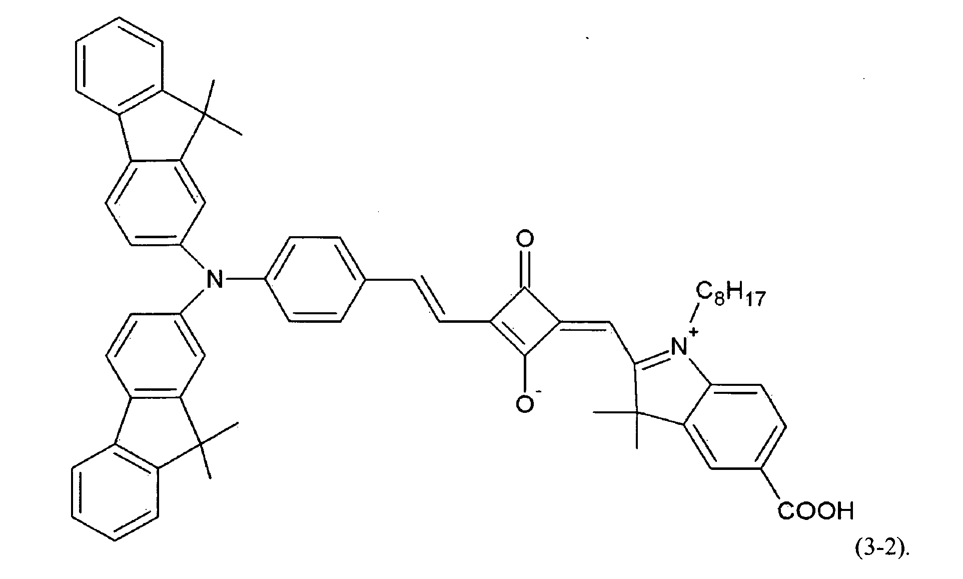

At least one of the above and other features and advantages may also be realized by providing a dye-sensitized solar cell including a first electrode including a conductive transparent substrate, a light absorption layer on one side of the first electrode, a second electrode facing the one side of the first electrode, and an electrolyte between the first electrode and the second electrode, wherein the light absorption layer includes a semiconductor particulate and the dye for a dye-sensitized solar cell as shown above.
The light absorption layer may further include at least one additive represented by Formula 4:

Z-COOH (4)

wherein Z is one of a hydrogen, a hydroxy, a halogen, a nitro, a cyano, a carboxyl, a substituted or unsubstituted amino, a substituted or unsubstituted acyl, a substituted or unsubstituted acyloxy, a substituted or unsubstituted alkyl, a substituted or unsubstituted cycloalkyl, a substituted or unsubstituted haloalkyl, a substituted or unsubstituted alkylsulfonyl, a substituted or unsubstituted arylsulfonyl, a substituted or unsubstituted alkylthio, a substituted or unsubstituted alkoxy, a substituted or unsubstituted alkoxysulfonyl, a substituted or unsubstituted alkoxycarbonyl, a substituted or unsubstituted aryl, a substituted or unsubstituted aryloxy, a substituted or unsubstituted alkenyl, a substituted or unsubstituted arylalkyl, or a substituted or unsubstituted heterocyclic group.

The additive may be one of deoxycholic acid, phenylpropionic acid, dodecylmalonic acid, and dodecylphosphonic acid.

The additive may be included in an amount of 100 to 3,000 parts by weight, based on 100 parts by weight of the dye for a dye-sensitized solar cell.

The light absorption layer may have a thickness of 25 µm or less.

### BRIEF DESCRIPTION OF THE DRAWINGS

The above and other features and advantages will become more apparent to those of ordinary skill in the art by describing in detail exemplary embodiments with reference to the attached drawings, in which:

FIG. 1 illustrates a schematic view of a dye-sensitized solar cell according to an embodiment;

FIG. 2 illustrates a graph showing incident photon-to-current efficiency (IPCE) according to wavelength for the dye-sensitized solar cell according to Example 1;

FIG. 3 illustrates a graph showing incident photon-to-current efficiency (IPCE) according to wavelength for the dye-sensitized solar cell according to Comparative Example 1; and

FIG. 4 illustrates a graph showing incident photon-to-current efficiency (IPCE) according to wavelength for the dye-sensitized solar cell according to Comparative Example 2.

### DETAILED DESCRIPTION

Example embodiments will now be described more fully hereinafter with reference to the accompanying drawings.

In the drawing figures, the dimensions of layers and regions may be exaggerated for clarity of illustration. It will also be understood that when a layer or element is referred to as being "on" another layer or substrate, it can be directly on the other layer or substrate, or intervening layers may also be present. Further, it will be understood that when a layer is referred to as being "under" another layer, it can be directly under, and one or more intervening layers may also be present. In addition, it will also be understood that when a layer is referred to as being "between" two layers, it can be the only layer between the two layers, or one or more intervening layers may also be present. Like reference numerals refer to like elements throughout. As used herein, the terms "a" and "an" are open terms that may be used in conjunction with singular items or with plural items.

As used herein, when specific definition is not provided, the term "alkyl" refers to a C₁ to C₃₀ alkyl and, in an implementation, a C₁ to C₂₀ an alkyl. The term "cycloalkyl" refers to a C₃ to C₃₀ cycloalkyl and, in an implementation, a C₃ to C₂₀ cycloalkyl. The term "haloalkyl" refers to a C₁ to C₃₀ haloalkyl and, in an implementation, a C₁ to C₂₀ haloalkyl. The term "alkylsulfonyl" refers to a C₁ to C₃₀ alkylsulfonyl and, in an implementation, a C₁ to C₂₀ an alkylsulfonyl. The term "arylsulfonyl" refers to a C₆ to Coo an arylsulfonyl and, in an implementation, a C₆ to C₂₀ arylsulfonyl. The term "an alkylthio" refers to a C₁ to C₃₀ alkylthio and, in an implementation, C₁ to C₂₀ an alkylthio. The term "alkoxy" refers to a C₁ to C₃₀ alkoxy and, in an implementation, a C₁ to C₂₀ alkoxy. The term "haloalkoxy" refers to a C₁ to C₃₀ haloalkoxy and, in an implementation, a C₁ to C₂₀ haloalkoxy. The term "alkoxysulfonyl" refers to a C₁ to C₃₀ alkoxysulfonyl and, in an implementation, a C₁ to C₂₀ alkoxysulfonyl. The term "alkoxycarbonyl" refers to a C₂ to C₃₀ alkoxycarbonyl and, in an implementation, a C₂ to C₂₀ alkoxycarbonyl. The term "acyl" refers to a C₁ to C₃₀ acyl and, in an implementation, a C₁ to C₂₀ acyl. The term "acyloxy" refers to a C₁ to C₃₀ acyloxy and, in an implementation, a C₁ to C₂₀ acyloxy. The term "aryl" refers to a C₆ to C₃₀ aryl and, in an implementation, a C₆ to C₂₀ aryl. The term "heteroaryl" refers to a C₆ to C₃₀ heteroaryl and, in an implementation, a C₆ to C₂₀ heteroaryl. The term "aryloxy" refers to a C₆ to C₃₀ aryloxy and, in an implementation, a C₆ to C₂₀ aryloxy. The term "alkenyl" refers to a C₂ to C₃₀ alkenyl and, in an implementation, a C₂ to C₂₀ alkenyl. The term "alkynyl" refers to a C₂ to C₃₀ alkynyl and, in an implementation, a C₂ to C₂₀ alkynyl. The term "arylalkyl" refers to a C₆ to C₃₀ arylalkyl and, in an implementation, a C₆ to C₂₀ arylalkyl. The term "heterocyclic group" refers to a C₂ to C₃₀ heterocyclic group and, in an implementation, a C₂ to C₂₀ heterocyclic group. The term "alkylene" refers to a C₁ to C₃₀ alkylene and, in an implementation, a C₁ to C₂₀ alkylene. The term "cycloalkylene" refers to a C₃ to C₃₀ cycloalkylene and, in an implementation, a C₃ to C₂₀ cycloalkylene. The term "alkenylene" refers to a C₂ to C₃₀ alkenylene and, in an implementation, a C₂ to C₂₀ alkenylene. The term "arylene" refers to a C₆ to C₃₀ arylene and, in an implementation, a C₆ to C₂₀ arylene.

As used herein, when specific definition is not provided, the term "aliphatic group" refers to a C₁ to C₃₀ alkyl, a C₂ to C₃₀ alkenyl, or a C₂ to C₃₀ alkynyl. The term "alicyclic group" refers to a C₃ to C₃₀ cycloalkyl, a C₃ to C₃₀ cycloalkenyl, or a C₃ to C₃₀ cycloalkynyl. The term "aromatic group" refers to a C₆ to C₃₀ aryl.

As used herein, when specific definition is not provided, the term "substituted" refers to one substituted with a substituent including at least one of hydroxy, a halogen, a nitro, a cyano, an amino, a carboxyl, a sulfonyl, an alkyl, a cycloalkyl, an alkoxy, a haloalkyl, a haloalkoxy, an acyl, an acyloxy, an alkylsulfonyl, an arylsulfonyl, an alkylthio, an alkoxysulfonyl, an alkoxycarbonyl, an aryl, an aryloxy, an alkenyl, an arylalkyl, and a heterocyclic group instead of hydrogen of a compound or a functional group. As used herein, when specific definition is not provided, the term "heterocyclic group" refers to a substituted or unsubstituted C₂ to C₃₀ cyclo alkyl, a substituted or unsubstituted C₂ to C₃₀ cycloalkenyl, a substituted or unsubstituted C₂ to C₃₀ cycloalkynyl, or a substituted or unsubstituted C₂ to C₃₀ heteroaryl that includes 1 to 3 heteroatoms including at least one of O, S, N, P, and Si in one ring.

As used herein, the symbol "*" refers to a portion to be linked to the same or different atom or chemical formula.

As used herein, when specific definition is not provided, the term "long wavelength region" refers to a range of wavelengths from a visible ray region to an infrared region, a wavelength region of about 400 nm to about 850 nm, or a wavelength region of about 550 nm to about 750 nm.

A dye-sensitized solar cell is an electrochemical solar cell that includes photosensitive dye molecules, which absorb light and produce electron-hole pairs, and a transition metal oxide, which transfers the produced electrons. The dye sensitized solar cell may use nano titanium oxide, e.g., anatase titanium oxide, which was described by Michael Grätzel et al. of the Swiss Federal Institute of Technology, Lausanne (EPFL), Switzerland, in 1991; see for instance a more recent work published in JACS (30/29, 9202 (2008). The dye sensitized solar cell may be produced at a low cost, and because it uses a transparent electrode, there may be an advantage in that it may be applied to external glass walls of a building or a glass greenhouse.

During driving of a dye-sensitized solar cell, photocharges may be generated by optical energy. In general, the photocharges may be generated by dye materials. The dye materials may be excited by absorbing light transmitted through a conductive transparent substrate.

For the dye materials, metal composites, e.g., a mono, bis, or tris(substituted 2,2'-bipyridine) complex salts of ruthenium have typically been used. However, such metal composites have a low efficiency because electrons excited by light may quickly return to a ground state. In order to increase efficiency, metal composites linked with various electron transporting materials through covalent bonds have been considered. However, linking the electron transporting materials through covalent bonds may require complicated processes.

Typical dye sensitized solar cells may have a limitation in application for practical use due to low photoelectric conversion efficiency. Therefore, new technology that improves the photoelectric conversion efficiency, e.g., the dye sensitized solar cell of an embodiment, is desirable.

According to an embodiment of the present invention, a compound for use as a dye for a dye-sensitized solar cell (hereinafter, "dye") includes a squaraine unit having a double bond at an end thereof. The compound also includes, linked to the end of the squaraine unit through the double bond, a functional group represented by Formula 5. According to a first alternative, the compound includes, linked to the end of the squaraine unit through the double bond, a substituted or unsubstituted benzofuran including a functional group linked to the ring oxygen (O). According to a second alternative, the compound includes, linked to the end of the squaraine unit through the double bond, a substituted or unsubstituted benzothiophene including a functional group linked to the ring sulfur (S).

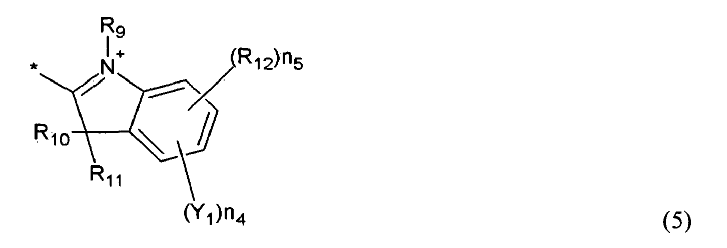

In Chemical Formula 5, R₉ to R₁₂ are each independently hydrogen, a substituted or unsubstituted aromatic group, a substituted or unsubstituted heterocyclic group, a substituted or unsubstituted aliphatic group, or a substituted or unsubstituted alicyclic group, Y₁ is an acidic functional group or a hydroxy group, n₄ is an integer of 1 to 4, n₅ is an integer of 0 to 3, and the sum of n₄+n₅ is 4 or less. In an implementation, the sum of n₄+n₅ may be 4, 3, 2, or 1. In another implementation, n₅ may be an integer of 1 to 3.

The compound is applicable as a dye, and may thereby improve short-circuit current and photoelectric conversion efficiency of a dye-sensitized solar cell, in addition to suppressing dark current generation. As described above, the compound may also include, linked to the end of the squaraine unit through the double bond, a substituted or unsubstituted benzofuran including a functional group linked to the ring oxygen (O), or a substituted or unsubstituted benzothiophene including a functional group linked to the ring sulfur (S). The functional groups linked to the ring oxygen (O) or ring sulfur (S) include, a substituted or unsubstituted aromatic group, a substituted or unsubstituted heterocyclic group, a substituted or unsubstituted aliphatic group, and a substituted or unsubstituted alicyclic group.

The dye is represented by Formula 1, Formula 2, or a combination thereof.

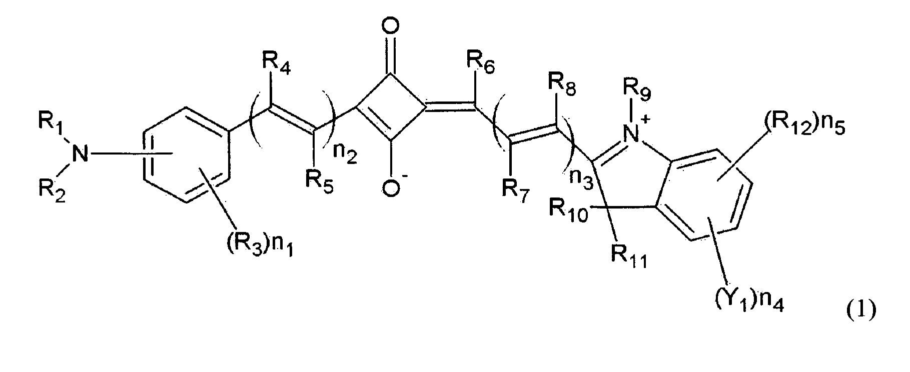

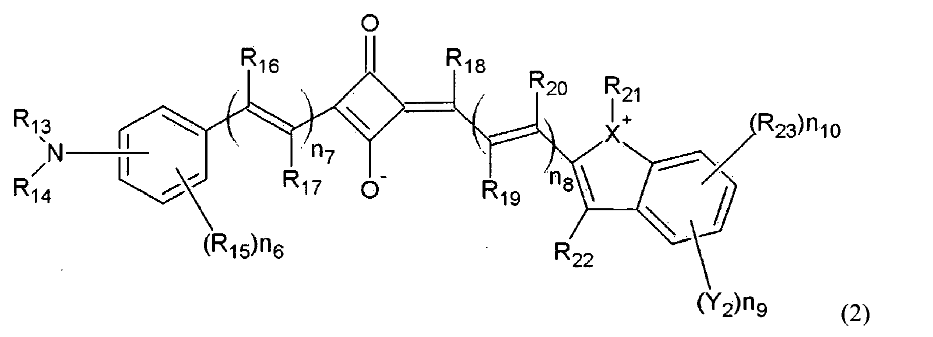

In Formulae 1 and 2, R₁ to R₃, R₉ to R₁₂, R₁₃ to R₁₅, and R₂₁ to R₂₃ may each independently be hydrogen, a substituted or unsubstituted aromatic group, a substituted or unsubstituted heterocyclic group, substituted or unsubstituted aliphatic group, or a substituted or unsubstituted alicyclic group. In Formulae 1 and 2, R₄ to R₈ and R₁₆ to R₂₀ may each independently be hydrogen or a substituted or unsubstituted aliphatic group. In Formula 2, X may be oxygen (O) or sulfur (S). In Formulae 1 and 2, Y₁ and Y₂ may each independently be an acidic functional group or a hydroxy group. In Formulae 1 and 2, n₁ and n₆ may each independently be integers of 0 to 4, n₂ and n₇ may each independently be integers of 1 to 4, n₃ and n₈ may each independently be integers of 0 to 2, n₄ and n₉ may each independently be integers of 1 to 4, n₅ and n₁₀ may each independently be integers of 0 to 3, the sum of n₄+n₅ may be 4 or less, and the sum of n₉+n₁₀ may be 4 or less. In an implementation, the sum of n₉+n₁₀ may be 4, 3, 2, or 1. In another implementation, n₁ and n₆ may each independently be integers of 1 to 4. In yet another implementation, n₅ and n₁₀ may each independently be integers of 1 to 3.

In an implementation, R₁, R₂, R₁₃, and R₁₄ may each independently be a substituted or unsubstituted C₉ to C₃₀ aromatic group, a substituted or unsubstituted C₂ to C₃₀ heterocyclic group, a substituted or unsubstituted C₁₃ to C₃₀ aliphatic group, or a substituted or unsubstituted C₃ to C₃₀ alicyclic group. In another implementation, R₁, R₂, R₁₃, and R₁₄ may each independently be a substituted or unsubstituted C₉ to C₂₀ aromatic group, a substituted or unsubstituted C₂ to C₂₀ heterocyclic group, a substituted or unsubstituted C₁₃ to C₂₀ aliphatic group, or a substituted or unsubstituted C₃ to C₂₀ alicyclic group.

The aromatic group may include, e.g., phenyl, naphthyl, xylyl, anthryl, phenanthryl, naphthacenyl, pyrenyl, biphenylyl, terphenylyl, tolyl, fluorenyl, indenyl, perylenyl, and the like.

The heterocyclic group may include, e.g., thiazolyl, benzothiazolyl, naphtothiazolyl, benzoxazolyl, naphtoxazolyl, imidazolyl, benzoimidazolyl, naphtoimidazolyl, thiazolyl, pyrrolyl, pyrazinyl, pyridyl, indolyl, isoindolyl, furyl, benzofuryl, isobenzofuryl, quinolyl, isoquinolyl, quinoxalinyl, carbazolyl, phenanthridinyl, acridinyl, phenanthrolinyl, phenazinyl, phenothiazinyl, phenoxazinyl, oxazolyl, oxadiazolyl, furazanyl, thienyl, and the like.

In another implementation, at least one of R₁, R₂, R₁₃, and R₁₄ may be a substituted or unsubstituted fluorenyl group.

In another implementation, at least one of R₁ and R₂ and at least one of R₁₃ and R₁₄ may be a substituted or unsubstituted fluorenyl group.

R₉ and R₂₁ may each independently be a substituted or unsubstituted C₁ to C₁₅ alkyl. In an implementation, R₉ and R₂₁ may be octyl.

Y₁ and Y₂ may each independently be a carboxyl group, a sulfonic acid group, a phosphoric acid group, or a hydroxy group.

R₄ to R₈ and R₁₆ to R₂₀ may each independently be hydrogen or a substituted or unsubstituted C₁ to C₁₅ aliphatic group.

In an implementation, the dye may be a compound represented by Formula 3-1.

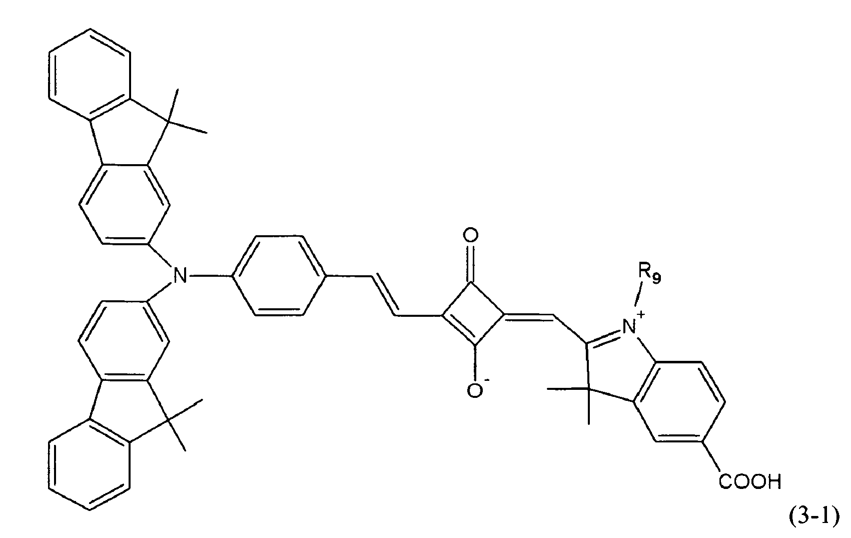

In Formula 3-1, R₉ may be covalently bound to the ring nitrogen and may be, e.g., hydrogen, a substituted or unsubstituted aromatic group, a substituted or unsubstituted heterocyclic group, substituted or unsubstituted aliphatic group, or a substituted or unsubstituted alicyclic group. In an implemetation, R₉ may be a linear alkyl group.

In an implementation, the dye may be a compound represented by Formula 3-2.

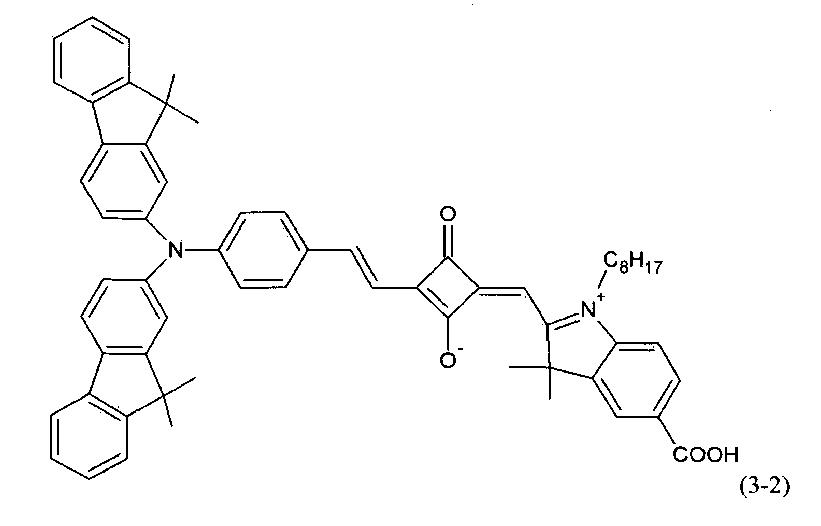

The dye includes an electron donor and electron acceptor linked to the squaraine unit. The squaraine unit acts as a strong electron transporter to lower a LUMO (lowest unoccupied molecular orbital) level of the compound, thereby enabling the dye to efficiently absorb light in a long wavelength region. The squaraine unit may include a hydroxyl (OH) to act as an auxiliary anchor for adsorbing onto semiconductor particulates.

The dye includes an electron acceptor linked to the squaraine unit. The electron acceptor may include a functional group such as a functional group represented by Formula 5 having an acidic functional group or a hydroxyl group. In another implementation, the electron acceptor may include a functional group including a substituted or unsubstituted benzofuran having an acidic functional group or a hydroxyl group. In another implementation, the electron acceptor may include a functional group including a substituted or unsubstituted benzothiophene having an acidic functional group or a hydroxyl group. Thus, the dye may efficiently absorb a light in a long wavelength region. The acidic functional group or hydroxyl may act as an electron acceptor and anchor. The functional group linked to nitrogen (N) of the functional group represented by Formula 5, the functional group linked to oxygen (O) of the benzofuran, and the functional group linked to sulfur (S) of the benzothiophene should be hydrophobic and bulky to protect the nitrogen (N), oxygen (O), and sulfur (S) from attack of triiodide ions (I₃⁻), and thereby suppress dark current generation.

In the dye, the electron donor, squaraine unit, and electron acceptor are linearly linked to each other. Thus, charge separation can be easily realized and charges can be present in an exited state for a long time, resulting in improved photoelectric conversion efficiency.

Therefore, the dye according to an embodiment exhibits high efficiency when exposed to light of a long wavelength region.

The dye may be applicable to various dye-sensitized solar cells. The dye is an easily purified organic compound and exhibits a high absorbance coefficient in a long wavelength region, as compared to a conventional dye. Accordingly, the dye of an embodiment is applicable to a thin film solar cell. The dye may be a transparent dye to absorb light in a long wavelength region including, e.g., an infrared region, to be applicable to a solar cell for buildings. The dye may be mixed with an organic dye to absorb a light in another wavelength region to be usable in a tandem solar cell. In this case, photoelectric conversion efficiency may be even more improved.

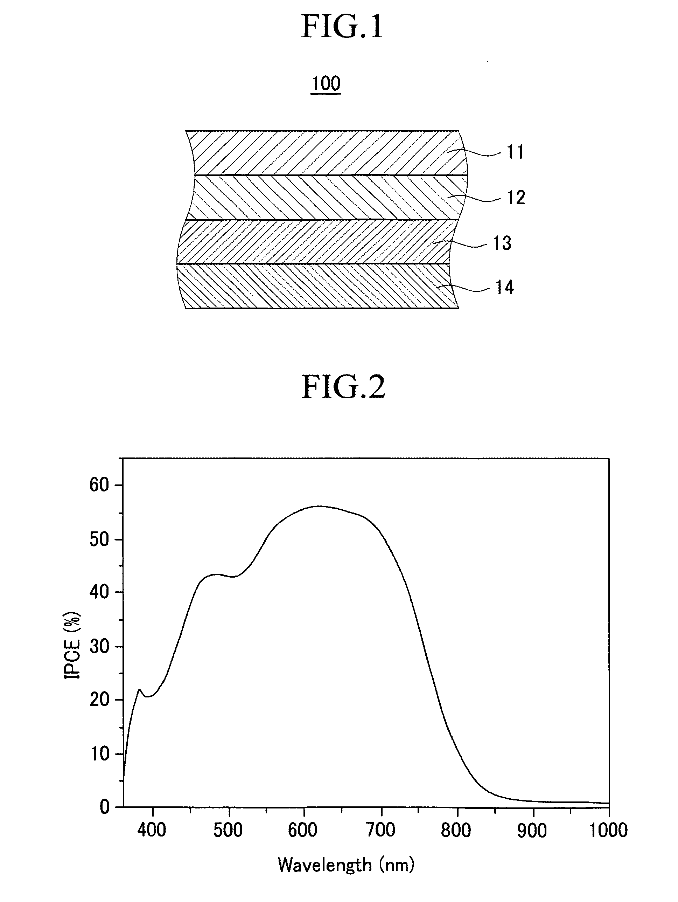

The dye-sensitized solar cell is described with reference to FIG. 1. FIG. 1 illustrates a cross-sectional view of a structure of a dye-sensitized solar cell 100 in accordance with an embodiment.

Referring to FIG. 1, the dye-sensitized solar cell 100 may have, e.g., a sandwich structure where two plate-type transparent electrodes, which may include a working electrode 11 and a counter electrode 14, respectively, facing each other. One side of one transparent electrode of the two transparent electrodes 11 and 14, e.g., the working electrode 11, may include a light absorption layer 12. The light absorption layer 12 may include a semiconductor particulate and the dye of an embodiment adsorbed on the semiconductor particulate. The electrons of the dye may be excited by absorbing light in the long wavelength region. A space between the two electrodes 11 and 14 may be filled with an electrolyte 13 for an oxidation-reduction reaction.

When light enters the dye-sensitized solar cell 100, dye molecules in the light absorption layer 12 may absorb photons. The dye molecules that have absorbed the photons may be excited from a ground state, to generate excitons (electron-hole pairs). The electrons may be injected into a conduction band of the semiconductor particulate. The injected electrons may be transferred from the light absorbing layer 12 to the working electrode 11 through the interface and then may be transferred to the counter electrode 14 through an external circuit. The dye that is oxidized as a result of the electron transfer may be reduced by an oxidation-reduction reaction in the electrolyte 13. The oxidized ions may be involved in a reduction reaction with electrons that have arrived at the interface of the counter electrode 14 to achieve charge neutrality. The dye sensitized solar cell 100 may be operated as described above.

The working electrode 11 may include a transparent substrate and a conductive layer, e.g., a transparent conductive layer, on the transparent substrate. The transparent substrate may be, e.g., a plastic substrate or a glass substrate. The glass substrate and the plastic substrate may have thereon a layer formed using, e.g., indium tin oxide (ITO), fluorine tin oxide (FTO), ZnO-(Ga₂O₃ or Al₂O₃), tin oxide (SnO₂), and/or zinc oxide. In an implementation, SnO₂ may be used because it has excellent conductivity, transparency, and heat resistance. In another implementation, ITO may be used because it has low production costs. The plastic substrate may include, e.g., polyethylene terephthalate (PET), polyethylene naphthalate (PEN), polycarbonate (PC), polypropylene (PP), polyimide (PI), and/or triacetylcellulose (TAC). The working electrode 11 may be doped with a doping material including, e.g., Ti, In, Ga, and/or Al.

The light absorption layer 12 may include a semiconductor particulate and the dye of an embodiment. The dye of an embodiment may be adsorbed on the semiconductor particulate, and the electrons of the dye may be excited by the absorption of light in long wavelength regions.

The semiconductor particulate may include, e.g., silicon, metal oxide, and/or composite metal oxide having a perovskite structure. The semiconductor may be, e.g., an n-type semiconductor, in which electrons of the conduction band become a carrier by being optically excited and provide an anode current. In particular, the semiconductor particulate may include, e.g., Si, Ge, TiO₂, SnO₂, ZnO, WO₃, Nb₂O₅, and/or TiSrO₃. The TiO₂ may be anatase TiO₂.

The semiconductor particulate may have a large surface area to make the dye adsorbed onto the surface of the semiconductor particulate absorb more light. The semiconductor particulate may have an average particle diameter of, e.g., 50 nm or less. Maintaining the particle diameter at 50 nm or less may help ensure that a surface area ratio does not decrease and thereby decrease the light absorption amount of dye and deteriorate catalyst efficiency. In an implementation, the semiconductor particulate may have an average particle diameter of 15 nm to 25 nm.

The light absorption layer 12 may further include at least one additive represented by Formula 4 in order to, e.g., improve photoelectric conversion efficiency of a solar cell.

Z-COOH (4)

In Formula 4, Z may be hydrogen, a hydroxy, a halogen, a nitro, a cyano, a carboxyl, a substituted or unsubstituted amino, a substituted or unsubstituted acyl, a substituted or unsubstituted acyloxy, a substituted or unsubstituted alkyl, a substituted or unsubstituted cycloalkyl, a substituted or unsubstituted haloalkyl, a substituted or unsubstituted alkylsulfonyl, a substituted or unsubstituted arylsulfonyl, a substituted or unsubstituted alkylthio, a substituted or unsubstituted alkoxy, a substituted or unsubstituted alkoxysulfonyl, a substituted or unsubstituted alkoxycarbonyl, a substituted or unsubstituted aryl, a substituted or unsubstituted aryloxy, a substituted or unsubstituted alkenyl, a substituted or unsubstituted arylalkyl, or a substituted or unsubstituted heterocyclic group.

In an implementation, the additive is deoxycholic acid represented by Formula 6, phenylpropionic acid, dodecylmalonic acid, or dodecylphosphonic acid.

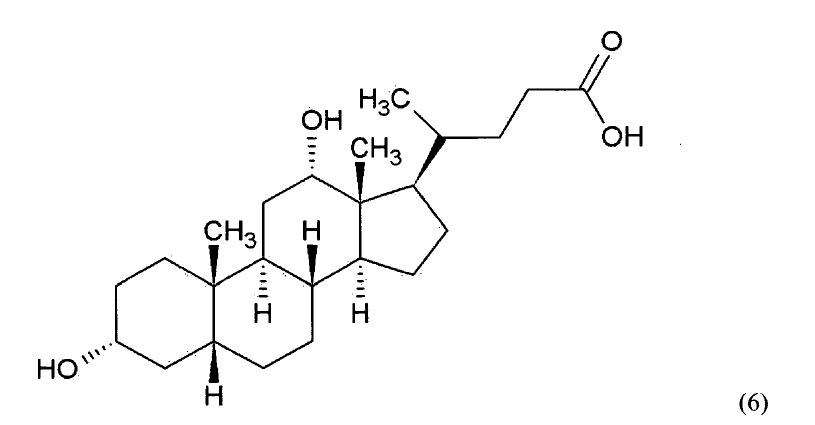

The additive may be included in an amount of 100 to 3,000 parts by weight, based on 100 parts by weight of the dye. Maintaining the amount of the additive at 100 to 3,000 parts by weight may help ensure that dye aggregation does not occur and the dye may be efficiently adsorbed on the semiconductor particulate. In an implementation, the additive may be included in an amount of 100 to 2,000 parts by weight, based on 100 parts by weight of the dye.

The light absorption layer 12 may have a thickness of 25 µm or less. Maintaining the thickness of the light absorption layer 12 at 25 µm or less may help ensure that serial resistance is lowered and electron transport efficiency to the working electrode 11 is improved, resulting in improved photoelectric conversion efficiency of a resultant solar cell. In an implementation, the thickness may be 1 to 25 µm. In another implementation, the thickness may be 5 to 25 µm.

The counter electrode 14 may be formed of any suitable material that has a conductive property. Even if the material is an insulating material, if a conductive layer is formed on a side facing the working electrode 11, it may be used as the counter electrode 14. The counter electrode 14 may include, e.g., Pt, Au, Ni, Cu, Ag, In, Ru, Pd, Rh, Ir, Os, C, and/or a conductive polymer.

The counter electrode 14 may be, e.g., a conductive layer including at least one of the above materials on a glass substrate or a plastic substrate. In another implementation, the glass substrate and the plastic substrate for the counter electrode 14 may have a conductive layer thereon including, e.g., indium tin oxide (ITO), fluorine tin oxide (FTO), ZnO-(Ga₂O₃ or Al₂O₃), tin oxide, and/or zinc oxide.

In order to improve redox catalyst efficiency, a side of the counter electrode 14 facing the working electrode 11 may have a micro structure to increase the surface area. For example, it may be desirable to form Pt or Au in a black state and to form carbon in a porous structure. Herein, the term 'black state' refers to a state not supported by a supporter. Particularly, platinum black may be formed by, e.g., performing anodic oxidation onto platinum or treating platinum with platinum chloride acid. The porous carbon may be formed by, e.g., sintering a carbon particulate or baking an organic polymer.

The electrolyte 13 may include, e.g., an iodide/triiodide pair that receives and transports electrons from the counter electrode 14 to the dye through an oxidation-reduction reaction. An open circuit voltage may be determined by a difference between an energy potential of the dye and a redox potential of the electrolyte. The electrolyte 13 may be uniformly dispersed between the working electrode 11 and counter electrode 14. The electrolyte 13 may also be impregnated inside the light absorption layer 12.

The electrolyte 13 may be a solution prepared by, e.g., dissolving iodine in acetonitrile. Alternatively, the electrolyte 13 may be any suitable substance having hole conductivity.

The dye sensitized solar cell 100 according to an embodiment may be fabricated by a method including, e.g., providing a first, i.e., working, electrode 11 including a conductive transparent substrate, forming a light absorption layer 12 including a semiconductor particulate and the dye of an embodiment on a side of the first electrode 11, fabricating a second, i.e., counter, electrode 14, arranging the first electrode 11 including the light absorption layer 12 and the second electrode 14 to face each other, filling an electrolyte 13 between the first electrode 11 and second electrode 14, and sealing the cell.

Hereinafter a process of forming the light absorption layer 12 is described in detail.

First, a conductive transparent substrate may be provided as the first electrode 11. A side of the conductive transparent substrate may be coated with a paste including a semiconductor particulate. Then, a heat treatment may be performed to thereby form a porous semiconductor particulate layer on the transparent substrate.

The properties of the paste may vary according to how the substrate is coated. The substrate may be coated with the paste using, e.g., a doctor blade or screen printing method. To form a transparent layer, e.g., a spin-coating or spraying method, may be used. Alternatively, a general wet coating method may be used. A binder may be added to the paste. The heat treatment may be carried out at about 400 °C to about 600 °C for about 30 minutes. If no binder is added, the heat treatment may be performed at a temperature of less than about 200 °C.

The porosity of the porous layer may be increased and maintained by adding a polymer to the porous semiconductor particulate layer and performing the heat treatment at about 400 °C to about 600 °C. The polymer may not leave organic material after the heat treatment. The polymer may include, e.g., ethylene cellulose (EC), hydroxy propyl cellulose (HPC), polyethylene glycol (PEG), polyethylene oxide (PEO), polyvinyl alcohol (PVA), and polyvinyl pyrrolidone (PVP). The polymer may have an appropriate molecular weight in consideration of the coating method and coating conditions. With an appropriate polymer added to the semiconductor particulate layer, a dispersion property as well as the porosity may be improved. Further, the layer may be better formed due to, e.g., an increased viscosity, and adhesiveness to the substrate may be improved.

A dye layer may be formed by, e.g., spraying a dye dispersion onto the semiconductor particulate layer or coating or dipping the semiconductor particulate layer with or in the dye dispersion to adsorb the dye on the semiconductor particulate. The dye dispersion may further include an additive including, e.g., compounds represented by Formula 6, in order to improve photoelectric efficiency of a resultant solar cell. The additive may be included at a concentration of about 0.3 mM to about 60 mM in the dye dispersion. The additive may be included in an amount of about 100 to about 3000 parts by weight, based on 100 parts by weight of the dye in the light absorption layer 12. Maintaining the concentration of the additive at about 0.3 mM to about 60 mM in the dye dispersion may help ensure that dye aggregation does not occur and dye adsorption efficiency is improved. In an implementation, the additive may be included at a concentration of about 5 mM to about 40 mM in the dye dispersion.

The dye may be naturally adsorbed on the semiconductor particulate when the first electrode 11 having the semiconductor particulate layer is dipped in the dye dispersion for, e.g., about 12 hours. The solvent dispersing the dye may be any suitable solvent including, e.g., acetonitrile, dichloromethane, and an alcohol-based solvent.

The dye dispersion may further include, e.g., an organic colorant of a variety of colors to further improve the long-wavelength light absorption and to further improve the light absorption efficiency of the dye. The organic colorant may include, e.g., coumarin and pheophorbide A, which is a kind of porphyrin.

After the dye layer is formed, preparation of the light absorption layer 12 may be completed by, e.g., washing out the dye that is not adsorbed through solvent washing.

The second electrode 14 may be prepared by forming a conductive layer including a conductive material on a conductive transparent substrate using, e.g., electroplating, electron beam deposition, or a physical vapor deposition (PVD) method such as sputtering.

The first electrode 11 and the second electrode 14 may be arranged such that the light absorption layer 12 faces the second electrode 14. Then, a space between the light absorption layer 12 and the second electrode 14 may be filled with the electrolyte 13 and sealed to complete the dye-sensitized solar cell 10.

The first electrode 11 and the second electrode 14 may be coupled to each other by using an adhesive agent. The adhesive agent may be, e.g., a thermoplastic polymer film, such as Surlyn produced by the DuPont Company. The thermoplastic polymer film may be placed between the two electrodes and heat and pressure may be applied to the electrodes. Alternatively, an epoxy resin or an ultraviolet (UV) ray curing material may be used as the adhesive agent. The adhesive agent may then be hardened after heat treatment or UV treatment.

The following examples illustrate the embodiments in more detail.

Preparation of dye for a dye-sensitized solar cell

The dye (3) was synthesized according to Reaction Scheme 1.

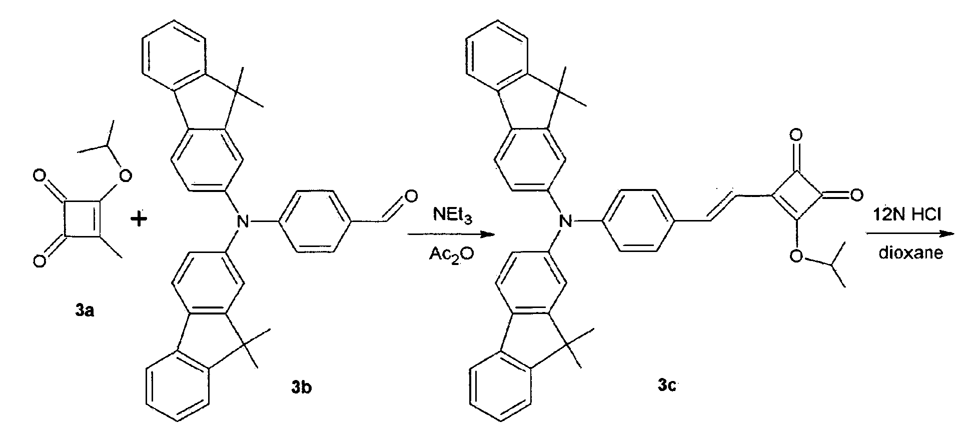

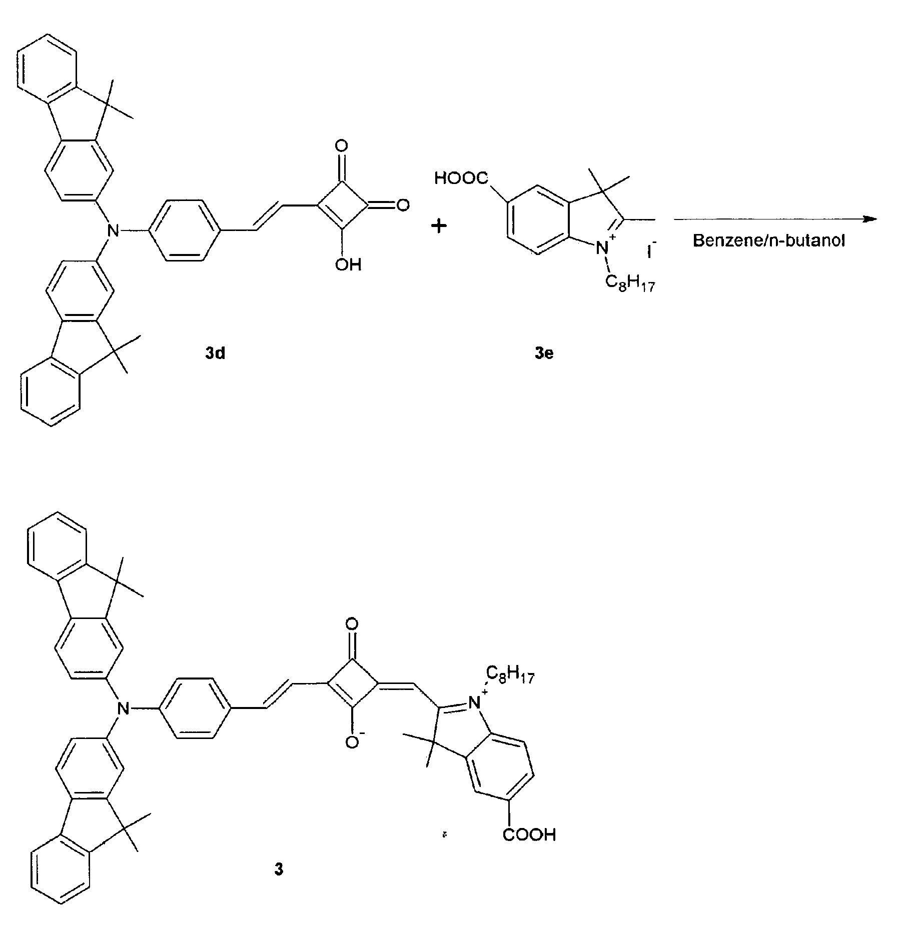

Referring to Reaction Scheme 1, a synthesis process of the dye (3) is described. First, a mixed solution including 4-(bis(9,9-dimethyl-9H-fluoren-2-yl)amino)benzaldehyde (3b) (1.91 g, 3.77 mmol), 3-isopropoxy-4-methylcyclobut-3-ene-1,2-dione (3a) (0.58 g, 3.77 mmol), triethylamine (NEt₃, 0.554 ml, 3.95 mmol), and acetic anhydride (Ac₂O) (0.369 ml 3.95 mmol) was refluxed for 8 hours and agitated. Then, the mixed solution was cooled to room temperature. A mixed solvent of water (30 ml) and dichloromethane (50 ml) was added to the cooled mixed solution to extract an organic layer. The extracted organic layer was subjected to a first drying using magnesium sulfate (MgSO₄) and then a second drying under vacuum. Then, the compound (3c) was separated using a silica gel chromatography.

A solution including the separated compound (3c), dioxane (2 ml), and hydrochloric acid (0.5 ml) was heated to 60 °C and then subjected to refluxing for 2 hours, agitating, and drying. A mixed solvent including water (30 ml) and dichloromethane (50 ml) was added the dried product to extract an organic layer. The extracted organic layer was subjected to a first drying using MgSO₄ and then a second drying under vacuum. Then, the compound (3d) was separated using a silica gel chromatography.

The compound (3d) (0.2 g, 0.316 mmol), and 5-carboxy-2,3,3-trimethyl-1-octyl-3H-indolium iodide (3e) (0.143 g, 0.316 mmol were dissolved in benzene (40 ml) and n-butanol (30 ml), followed by refluxing for 24 hours and agitating to prepare a mixed solution.

The mixed solution was dried using a rotary evaporator under vacuum to obtain a dried product. A mixed solvent including water (30 ml) and dichloromethane (50 ml) was added the dried product to extract an organic layer. The extracted organic layer was subjected to a first drying using MgSO₄ and then a second drying under vacuum. Then, the compound (3) was separated using silica gel chromatography.

Example 1: Fabrication of a dye-sensitized solar cell

A titanium dioxide dispersion solution including titanium dioxide particles with a particle diameter of 5 to 15 nm was applied to 1 cm² of an indium-doped tin oxide transparent conductor using a doctor blade method. A heat treatment was performed at 450 °C for 30 minutes to form a 18 µm-thick porous titanium dioxide layer. A 0.3 mM dye dispersion solution was prepared by dissolving a compound represented by Chemical Formula 3 in ethanol, and then a resulting dye dispersion solution was prepared by adding deoxycholic acid to be 10 mM in the 0.3 mM dye dispersion solution. The 18 µm-thick porous titanium oxide layer was maintained at 80 °C and dipped in the resulting dye dispersion solution to adsorb the dye for over 12 hours.

The dye-adsorbed porous titanium oxide layer was washed with ethanol and dried at room temperature to thereby form a first electrode with a light absorption layer thereon.

A second electrode was prepared by depositing a 200 nm-thick Pt layer on an indium-doped tin oxide transparent conductor by sputtering. Then, a fine hole was formed therein with a drill having a diameter of 0.75 mm.

A 60 µm-thick thermoplastic polymer film was disposed between the first electrode and the second electrode and pressure was applied to the first and second electrodes at 100 °C for 9 seconds to adhere the two electrodes. An oxidation-reduction electrolyte was injected through the fine hole in the second electrode and the fine hole was sealed using a cover glass and a thermoplastic polymer film to thereby fabricate a dye-sensitized solar cell. When the dye-sensitized solar cell was a 0.2 cm² cell, about 1 ml of the oxidation-reduction electrolyte was injected therein. The oxidation-reduction electrolyte was prepared by dissolving 1,2-dimethyl-3-hexylimidazolium iodide, 2-aminopyrimidine, LiI, and I₂ in an acetonitrile solvent. In the prepared oxidation-reduction electrolyte, the concentration of the 1,2-dimethyl-3-hexylimidazolium iodide was 0.62 M, that of the 2-aminopyrimidine was 0.5 M, that of the LiI was 0.1 M, and that of the I₂ was 0.05 M.

Comparative Example 1: Fabrication of a dye-sensitized solar cell

A titanium oxide dispersion solution including titanium oxide particles with an average particle diameter of 5 to 15 nm was applied to 1 cm² of an indium-doped tin oxide transparent conductor using a doctor blade method. Then, a heat treatment was performed at 450 °C for 30 minutes to thereby form a 18 µm-thick porous titanium oxide layer. A 0.3 mM dye dispersion solution was prepared by dissolving a compound represented by Chemical Formula 7 in ethanol, and then a resulting dye dispersion solution was prepared by adding deoxycholic acid to be 10 mM in the 0.3 mM dye dispersion solution. The 18 µm-thick porous titanium oxide layer was maintained at 80 °C and dipped in the resulting dye dispersion solution to adsorb the dye for over 12 hours.

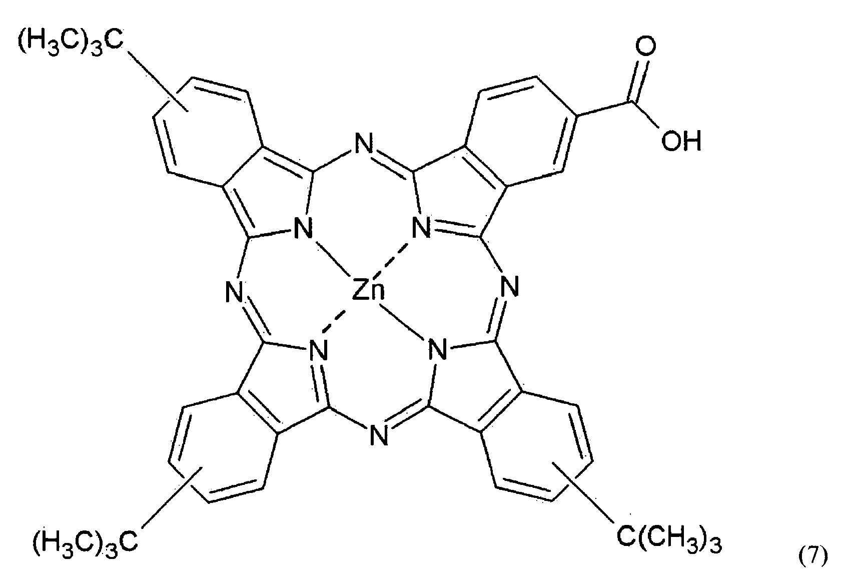

The dye-adsorbed porous titanium oxide layer was washed with ethanol and dried at room temperature to form a first electrode with a light absorption layer thereon.

A second electrode was prepared by depositing a 200 nm-thick Pt layer on an indium-doped tin oxide transparent conductor by sputtering. Then, a fine hole was formed therein with a drill having a diameter of 0.75 mm.

A 60 µm-thick thermoplastic polymer film was disposed between the first electrode and the second electrode. Pressure was then applied to the first and second electrodes at 100 °C for 9 seconds to adhere the two electrodes. An oxidation-reduction electrolyte was injected through the fine hole in the second electrode. The fine hole was then sealed using a cover glass and a thermoplastic polymer film to thereby fabricate a dye-sensitized solar cell. When the dye-sensitized solar cell was 0.2 cm² cell, about 1 ml of the oxidation-reduction electrolyte was injected therein. The oxidation-reduction electrolyte was prepared by dissolving 1,2-dimethyl-3-hexylimidazolium iodide, 2-aminopyrimidine, LiI, and I₂ in an acetonitrile solvent. In the prepared oxidation-reduction electrolyte, the concentration of the 1,2-dimethyl-3-hexylimidazolium iodide was 0.62 M, that of the 2-aminopyrimidine was 0.5 M, that of the LiI was 0.1 M, and that of the I₂ was 0.05 M.

Comparative Example 2: Fabrication of a dye-sensitized solar cell

A dye-sensitized solar cell was fabricated according to the same method as in Comparative Example 1, except that a compound represented Chemical Formula 8 was used for the dye.

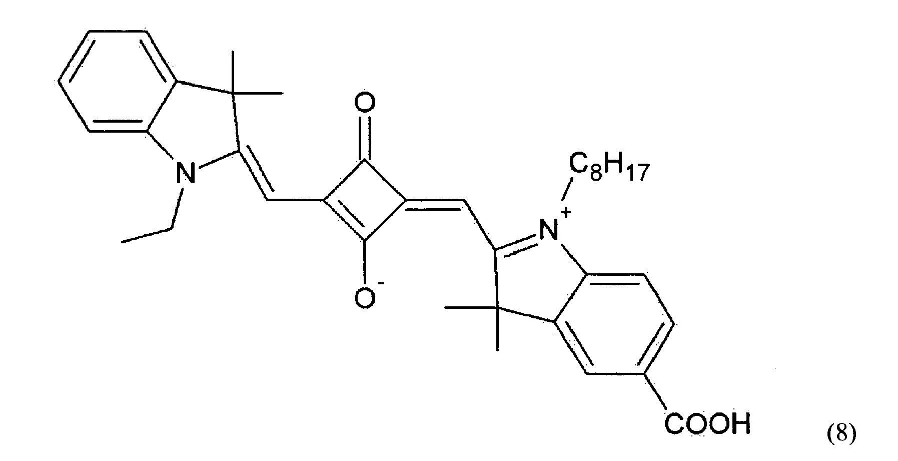

Experimental Example 1: Evaluation of dye-sensitized solar cells

Photocurrent voltages of the dye-sensitized solar cells according to Example 1 and Comparative Examples 1 and 2 were measured. The open-circuit voltage (Voc), current density (short-circuit current: Jsc), and a fill factor (FF) were calculated based on a curve line of the measured photocurrent voltages. From the results, solar cell efficiency was evaluated. The results are shown in Table 1.

A xenon lamp (Oriel, 01193), was used as a light source, and the solar condition (AM 1.5) of the xenon lamp was corrected by using a standard solar cell (Fraunhofer Institut Solare Energiesysteme, Certificate No. C-ISE369, Type of material: Mono-Si + KG filter).

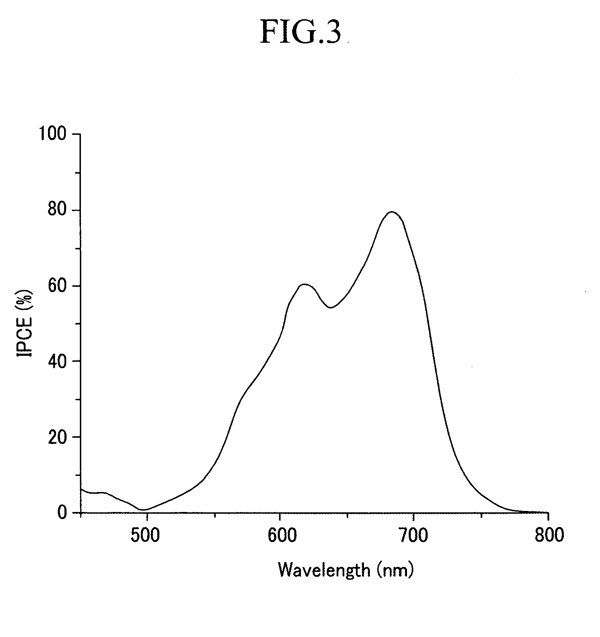

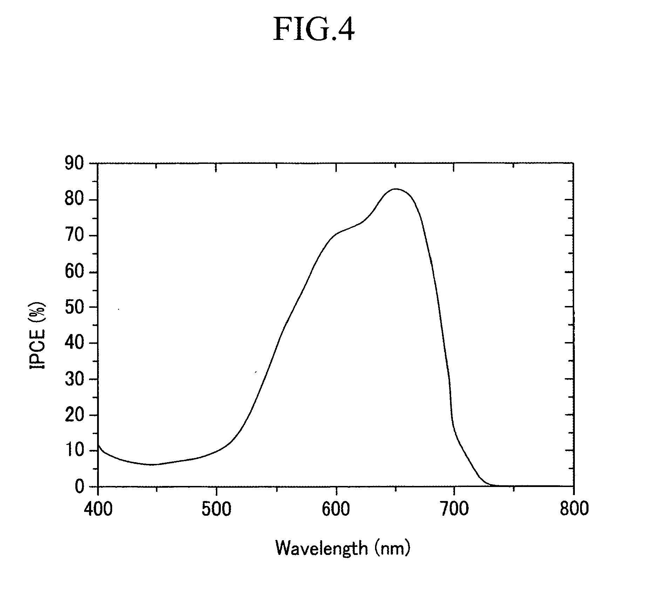

Photoelectric conversion efficiency (IPCE, incident photon to current efficiency) of the dye-sensitized solar cells according to Example 1, Comparative Example 1, and Comparative Example 2 were measured. Herein, the IPCE was evaluated by measuring intensity of photoelectric conversion values of the dye-sensitized solar cell at each wavelength region using SR-810 (PV measurements Inc.). The photoelectric conversion efficiency measurement results are illustrated in FIGS. 2, 3, and 4.

**Table 1**

| | Example 1 | Comparative Example 1 | Comparative Example 2 |
|---|---|---|---|
| Voc (V) | 0.493 | 0.617 | 0.603 |
| Jsc (mA/cm²) | 12.54 | 7.60 | 10.50 |
| FF | 70 | 75 | 71 |
| Efficiency (%) | 4.33 | 3.52 | 4.5 |

As shown in Table 1, current density (short-circuit current, Jsc) of the dye-sensitized solar cell of Example 1 was greater than those of the dye-sensitized solar cells according to Comparative Examples 1 and 2. As illustrated in FIGS. 2 to 4, the dye-sensitized solar cell of Example 1 absorbed light in a long wavelength region of about 400 nm to about 800 nm. On the contrary, the dye-sensitized solar cell of Comparative Example 1 absorbed light in a wavelength region of only about 600 nm to about 700 nm. The dye-sensitized solar cell of Comparative Example 2 absorbed light in a wavelength region of only about 550 nm to about 700 nm.

Therefore, the dye-sensitized solar cell of Example 1 absorbed light in an infrared ray (IR) region as well as a visible ray region to efficiently convert light energy into electrical energy. Accordingly, the current density (short-circuit current, Jsc) of the dye-sensitized solar cell according to Example 1 was greater than those of the dye-sensitized solar cell of Comparative Examples 1 and 2.

Also, as shown in Table 1, the overall photoelectric conversion efficiency of the dye-sensitized solar cell of Example 1 was greater than that of the dye-sensitized solar cell according to Comparative Example 1, and was equivalent to that of the dye-sensitized solar cell according to Comparative Example 2.

## Claims

1. A dye for a dye-sensitized solar cell represented by Formula 1, Formula 2, or a combination thereof: 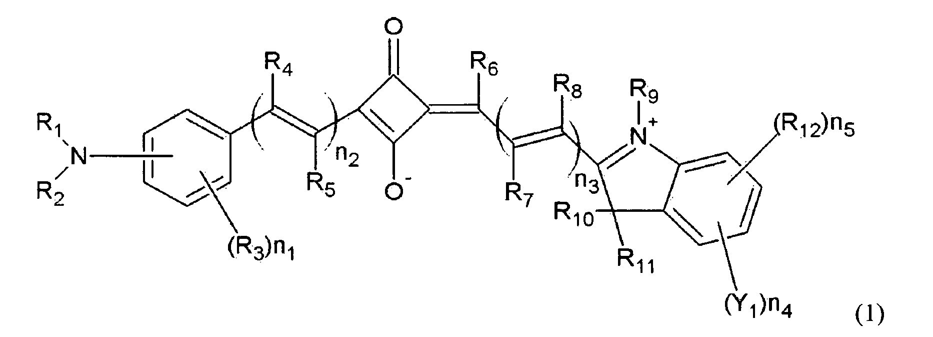 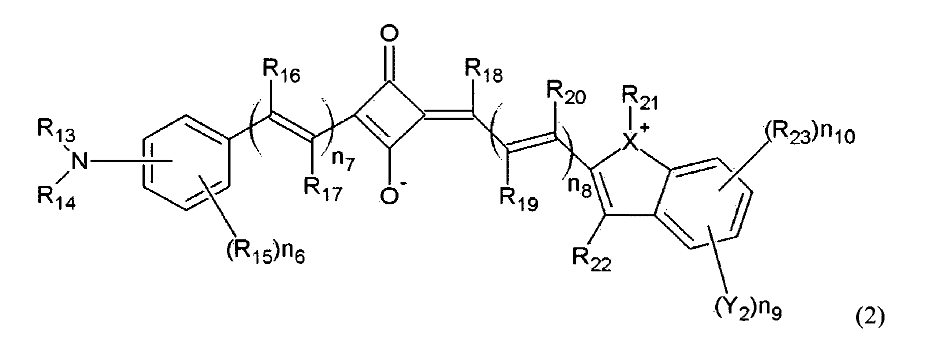 wherein, in Formulae 1 and 2:
R₁ to R₃, R₉ to R₁₂, R₁₃ to R₁₅, and R₂₁ to R₂₃ are each independently hydrogen, a substituted or unsubstituted aromatic group, a substituted or unsubstituted heterocyclic group, substituted or unsubstituted aliphatic group, or a substituted or unsubstituted alicyclic group,
R₄ to R₈ and R₁₆ to R₂₀ are each independently hydrogen or a substituted or unsubstituted aliphatic group,
X is O or S,
Y₁ and Y₂ are each independently an acidic functional group or a hydroxy group,
n₁ and n₆ are each independently integers of 0 to 4,
n₂ and n₇ are each independently integers of 1 to 4,
n₃ and n₈ are each independently integers of 0 to 2,
n₄ and n₉ are each independently integers of 1 to 4,
n₅ and n₁₀ are each independently integers of 0 to 3,
n₄+n₅ is an integer of 4 or less, and
n₉+n₁₀ is an integer of 4 or less.

2. The dye of claim 1, wherein R₁, R₂, R₁₃ and R₁₄ are each independently a substituted or unsubstituted C₉ to C₃₀ aromatic group, a substituted or unsubstituted C₂ to C₃₀ heterocyclic group, a substituted or unsubstituted C₁₃ to C₃₀ aliphatic group, or a substituted or unsubstituted C₃ to C₃₀ alicyclic group.

3. The dye of claim 2, wherein at least one of R₁, R₂, R₁₃ and R₁₄ is a substituted or unsubstituted fluorenyl group.

4. The dye of any one of the preceding claims, wherein R₉ and R₂₁ are each independently a substituted or unsubstituted C₅ to C₁₅ aliphatic group.

5. The dye of any one of the preceding claims, wherein Y₁ and Y₂ are each independently a carboxyl group, a sulfonic acid group, a phosphoric acid group, or a hydroxy group.

6. The dye of claim 1 or claim 4, wherein the dye is represented by Formula 3-1:

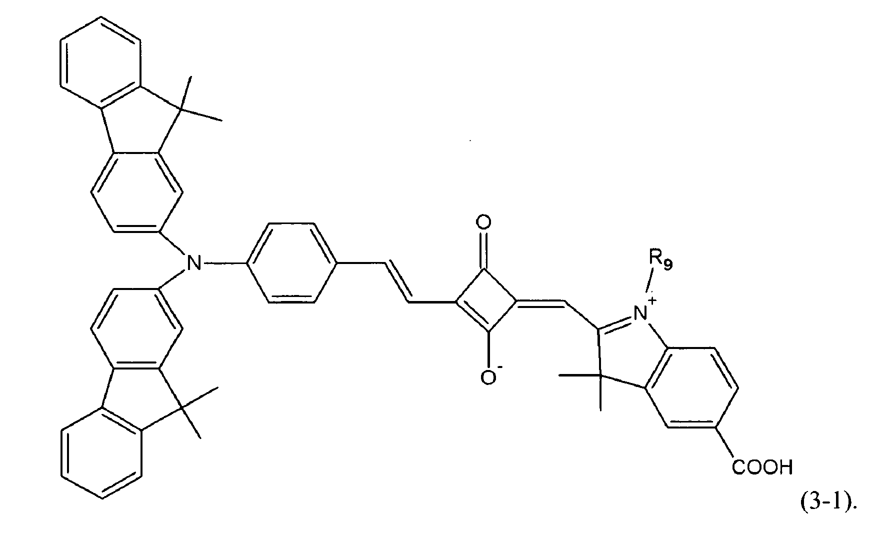

7. The dye of claim 6, wherein the dye is represented by Formula 3-2:

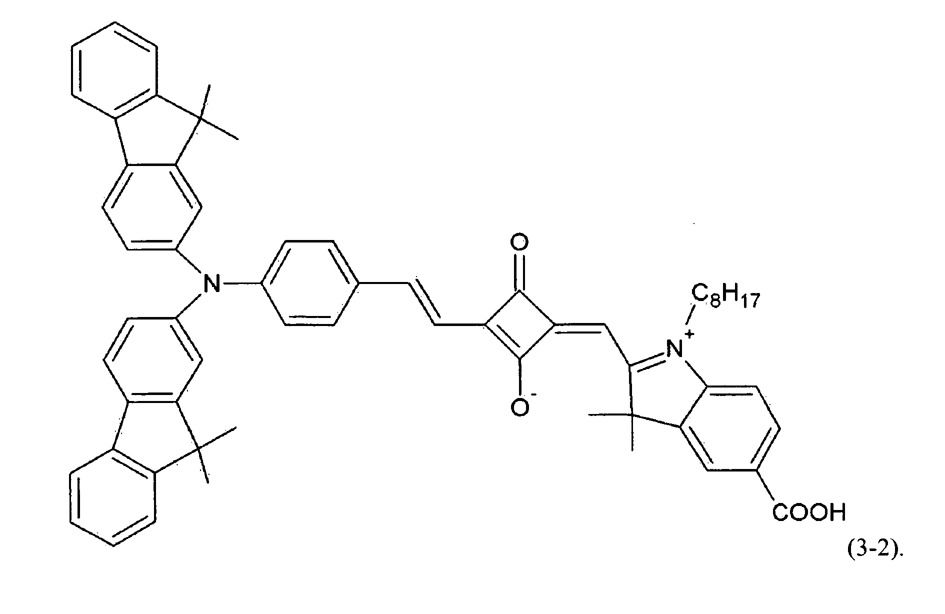

8. A dye-sensitized solar cell, comprising:
a first electrode including a conductive transparent substrate;
a light absorption layer on one side of the first electrode;
a second electrode facing the one side of the first electrode; and
an electrolyte between the first electrode and the second electrode,
wherein the light absorption layer includes a semiconductor particulate and the dye according to any one of claims 1 through 7.

9. The dye-sensitized solar cell of claim 8, wherein the light absorption layer further includes at least one additive represented by Formula 4:
Z-COOH (4)
wherein Z is one of a hydrogen, a hydroxy, a halogen, a nitro, a cyano, a carboxyl, a substituted or unsubstituted amino, a substituted or unsubstituted acyl, a substituted or unsubstituted acyloxy, a substituted or unsubstituted alkyl, a substituted or unsubstituted cycloalkyl, a substituted or unsubstituted haloalkyl, a substituted or unsubstituted alkylsulfonyl, a substituted or unsubstituted arylsulfonyl, a substituted or unsubstituted alkylthio, a substituted or unsubstituted alkoxy, a substituted or unsubstituted alkoxysulfonyl, a substituted or unsubstituted alkoxycarbonyl, a substituted or unsubstituted aryl, a substituted or unsubstituted aryloxy, a substituted or unsubstituted alkenyl, a substituted or unsubstituted arylalkyl, or a substituted or unsubstituted heterocyclic group.

10. The dye-sensitized solar cell of claim 9, wherein the additive is one of deoxycholic acid, phenylpropionic acid, dodecylmalonic acid, and dodecylphosphonic acid.

11. The dye-sensitized solar cell of claim 9 or 10, wherein the additive is included in an amount of 100 to 3,000 parts by weight, based on 100 parts by weight of the dye for a dye-sensitized solar cell.

12. The dye-sensitized solar cell of any one of claims 8 through 11, wherein the light absorption layer has a thickness of 25 µm or less.

## Patentansprüche

1. Farbstoff für eine farbstoffsensibilisierte Solarzelle, dargestellt durch Formel 1, Formel 2 oder deren Kombination: 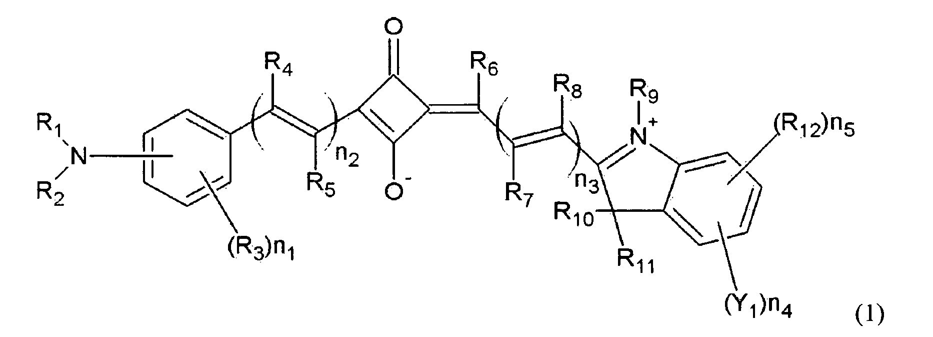 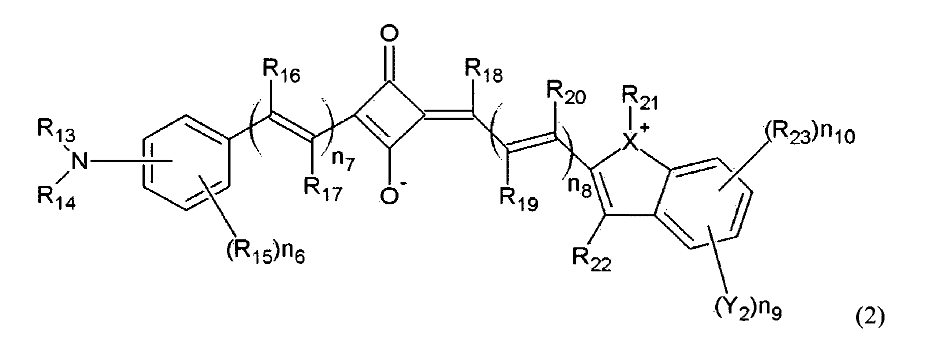 wobei in Formel 1 und 2:
R₁ bis R₃, R₉ bis R₁₂, R₁₃ bis R₁₅ und R₂₁ bis R₂₃ jeweils unabhängig voneinander Wasserstoff, eine substituierte oder unsubstituierte aromatische Gruppe, eine substituierte oder unsubstituierte heterozyklische Gruppe, eine substituierte oder unsubstituierte aliphatische Gruppe oder eine substituierte oder unsubstituierte alyzyklische Gruppe sind,
R₄ bis R₈ und R₁₆ bis R₂₀ jeweils unabhängig voneinander Wasserstoff oder eine substituierte oder unsubstituierte aliphatische Gruppe sind,
X O ist oder S,
Y₁ und Y₂ jeweils unabhängig voneinander eine saure funktionelle Gruppe oder eine Hydroxygruppe sind,
n₁ und n₆ jeweils unabhängig voneinander ganze Zahlen von 0 bis 4 sind,
n₂ und n₇ jeweils unabhängig voneinander ganze Zahlen von 1 bis 4 sind,
n₃ und n₈ jeweils unabhängig voneinander ganze Zahlen von 0 bis 2 sind,
n₄ und n₉ jeweils unabhängig voneinander ganze Zahlen von 1 bis 4 sind,
n₅ und n₁₀ jeweils unabhängig voneinander ganze Zahlen von 0 bis 3 sind,
n₄+n₅ eine ganze Zahl von 4 oder weniger ist, und
n₉+n₁₀ eine ganze Zahl von 4 oder weniger ist.

2. Farbstoff nach Anspruch 1, wobei R₁, R₂, R₁₃ und R₁₄ jeweils unabhängig voneinander eine substituierte oder unsubstituierte aromatische C₉ - bis C₃₀ -Gruppe, eine substituierte oder unsubstituierte heterozyklische C₂ - bis C₃₀ -Gruppe, eine substituierte oder unsubstituierte aliphatische C₁₃ - bis C₃₀ -Gruppe oder eine substituierte oder unsubstituierte alizyklische C₃ - bis C₃₀ -Gruppe sind.

3. Farbstoff nach Anspruch 2, wobei zumindest eines von R₁, R₂, R₁₃ und R₁₄ eine substituierte oder unsubstituierte Fluorenylgruppe ist.

4. Farbstoff nach einem der vorhergehenden Ansprüche, wobei R₉ und R₂₁ jeweils unabhängig voneinander eine substituierte oder unsubstituierte aliphatische C₅ - bis C₁₅ -Gruppe sind.

5. Farbstoff nach einem der vorhergehenden Ansprüche, wobei Y₁ und Y₂ jeweils unabhängig voneinander eine Carboxylgruppe, eine Sulfonsäuregruppe, eine Phosphorsäuregruppe oder eine Hydroxygruppe sind.

6. Farbstoff nach Anspruch 1 oder 4, wobei der Farbstoff durch Formel 3-1 dargestellt ist:

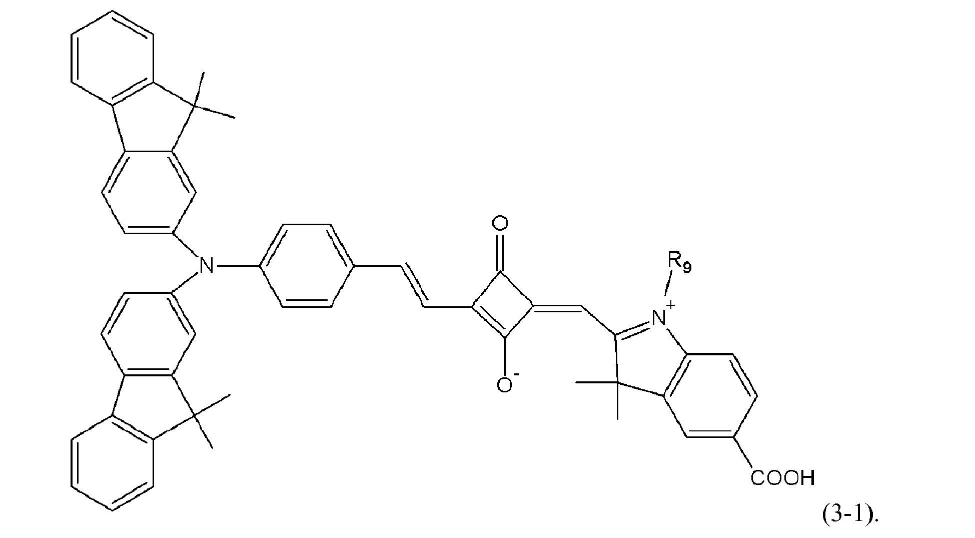

7. Farbstoff nach Anspruch 6, wobei der Farbstoff durch Formel 3-2 dargestellt ist:

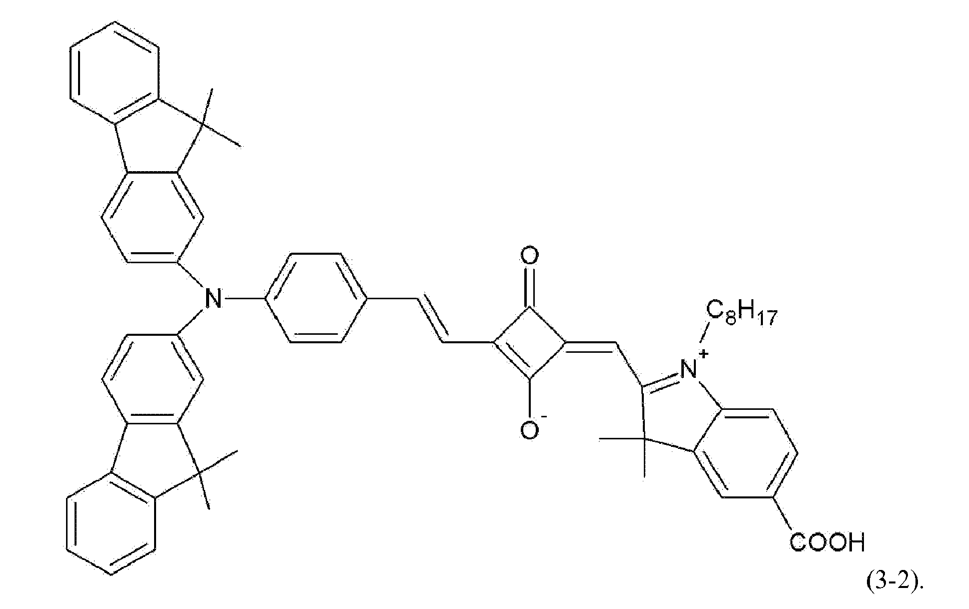

8. Farbstoffsensibilisierte Solarzelle, aufweisend:
eine erste Elektrode, die ein leitendes transparentes Substrat aufweist;
eine lichtabsorbierende Schicht auf einer Seite der ersten Elektrode;
eine zweite Elektrode, die der einen Seite der ersten Elektrode zugewandt ist; und
einen Elektrolyt zwischen der ersten Elektrode und der zweiten Elektrode,
wobei die lichtabsorbierende Schicht einen Halbleiterpartikel und den Farbstoff nach einem der Ansprüche 1 bis 7 aufweist.

9. Farbstoffsensibilisierte Solarzelle nach Anspruch 8, wobei die lichtabsorbierende Schicht weiterhin zumindest ein Additiv aufweist, das durch Formel 4 dargestellt ist:
Z-COOH (4)
wobei Z eines von einem Wasserstoff, einem Hydroxy, einem Halogen, einem Nitro, einem Cyano, einem Carboxyl, einem substituierten oder unsubstituierten Amino, einem substituierten oder unsubstituierten Acyl, einem substituierten oder unsubstituierten Acyloxy, einem substituierten oder unsubstituierten Alkyl, einem substituierten oder unsubstituierten Cycloalkyl, einem substituierten oder unsubstituierten Halogenalkyl, einem substituierten oder unsubstituierten Alkylsulfonyl, einem substituierten oder unsubstituierten Arylsulfonyl, einem substituierten oder unsubstituierten Alkylthio, einem substituierten oder unsubstituierten Alkoxy, einem substituierten oder unsubstituierten Alkoxysulfonyl, einem substituierten oder unsubstituierten Alkoxycarbonyl, einem substituierten oder unsubstituierten Aryl, einem substituierten oder unsubstituierten Aryloxy, einem substituierten oder unsubstituierten Alkenyl, einem substituierten oder unsubstituierten Arylalkyl oder einer substituierten oder unsubstituierten heterzozyklischen Gruppe ist.

10. Farbstoffsensibilisierte Solarzelle nach Anspruch 9, wobei das Additiv Desoxycholsäure, Phenylpropionsäure, Dodecylmalonsäure oder Dodecylphosphonsäure ist.

11. Farbstoffsensibilisierte Solarzelle nach Anspruch 9 oder 10, wobei das Additiv in einer Menge von 100 bis 3000 Gewichtsteilen, bezogen auf 100 Gewichtsteile des Farbstoffs für eine farbstoffsensibilisierte Solarzelle, enthalten ist.

12. Farbstoffsensibilisierte Solarzelle nach einem der Ansprüche 8 bis 11, wobei die lichtabsorbierende Schicht eine Dicke von 25 µm oder weniger aufweist.

## Revendications

1. Colorant pour une cellule solaire sensibilisée avec un colorant, représenté par la formule 1, la formule 2 ou une de leurs associations : 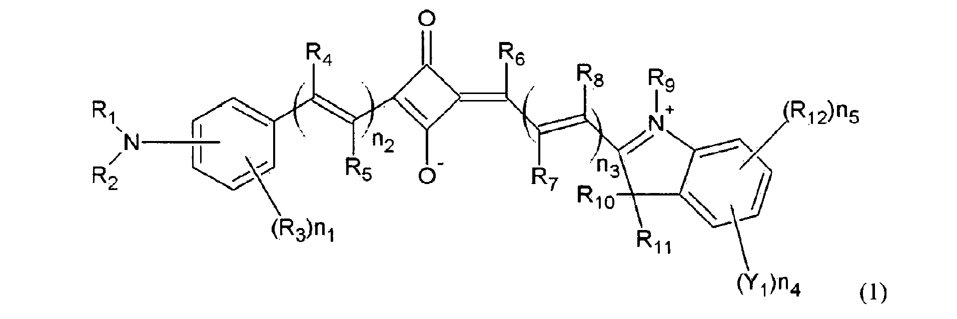 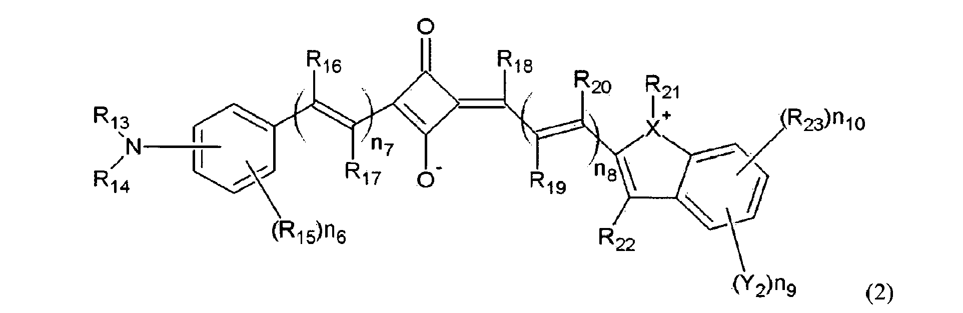 où, dans les formules 1 et 2 :
R₁ à R₃, R₉ à R₁₂, R₁₃ à R₁₅ et R₂₁ à R₂₃ représentent chacun indépendamment un atome d'hydrogène, un groupe aromatique substitué ou non substitué, un groupe hétérocyclique substitué ou non substitué, un groupe aliphatique substitué ou non substitué, un groupe alicyclique substitué ou non substitué,
R₄ à R₈ et R₁₆ à R₂₀ représentent chacun indépendamment un atome d'hydrogène ou un groupe aliphatique substitué ou non substitué,
X représente O ou S,
Y₁ et Y₂ représentent chacun indépendamment un groupe fonctionnel acide ou un groupe hydroxy,
n₁ et n₆ représentent chacun indépendamment des nombres entiers de 0 à 4,
n₂ et n₇ représentent chacun indépendamment des nombres entiers de 1 à 4,
n₃ et n₈ représentent chacun indépendamment des nombres entiers de 0 à 2,
n₄ et n₉ représentent chacun indépendamment des nombres entiers de 1 à 4,
n₅ et n₁₀ représentent chacun indépendamment des nombres entiers de 0 à 3,
n₄+n₅ représentent un nombre entier égal ou inférieur à 4, et
n₉+n₁₀ représentent un nombre entier égal ou inférieur à 4.

2. Colorant suivant la revendication 1, dans lequel R₁, R₂, R₁₃ et R₁₄ représentent chacun indépendamment un groupe aromatique en C₉ à C₃₀ substitué ou non substitué, un groupe hétérocyclique en C₂ à C₃₀ substitué ou non substitué, un groupe aliphatique en C₁₃ à C₃₀ substitué ou non substitué ou un groupe alicyclique en C₃ à C₃₀ substitué ou non substitué.

3. Colorant suivant la revendication 2, dans lequel au moins un de R₁, R₂, R₁₃ et R₁₄ représentent un groupe fluorényle substitué ou non substitué.

4. Colorant suivant l'une quelconque des revendications précédentes, dans lequel R₉ et R₂₁ représentent chacun indépendamment un groupe aliphatique en C₅ à C₁₅ substitué ou non substitué.

5. Colorant suivant l'une quelconque des revendications précédentes, dans lequel Y₁ et Y₂ représentent chacun indépendamment un groupe carboxyle, un groupe acide sulfonique, un groupe acide phosphorique ou un groupe hydroxy.

6. Colorant suivant la revendication 1 ou la revendication 4, ledit colorant étant représenté par la formule 3-1 :

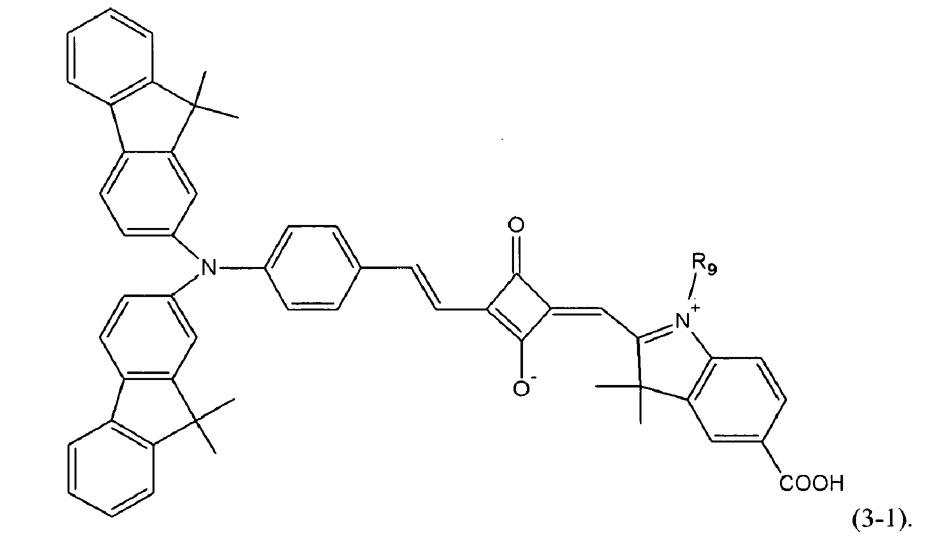

7. Colorant suivant la revendication 6, ledit colorant étant représenté par la formule 3-2 :

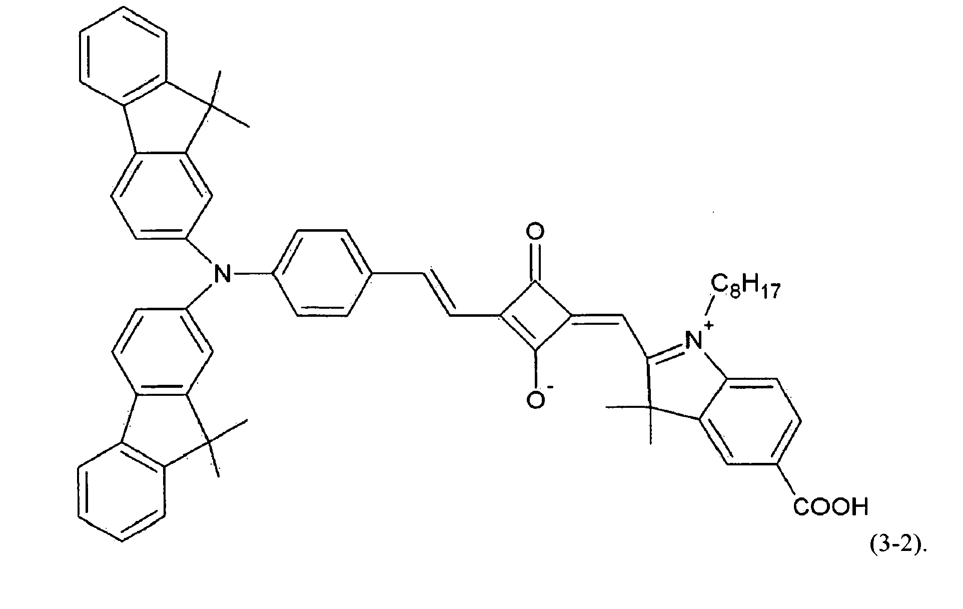

8. Cellule solaire sensibilisée avec un colorant, comprenant une première électrode comprenant un substrat transparent conducteur ;
une couche absorbant la lumière sur une face de la première électrode ;
une seconde électrode tournée du côté de la première électrode ; et
un électrolyte entre la première électrode et la seconde électrode,
dans laquelle la couche absorbant la lumière comprend un semi-conducteur en particules et le colorant suivant l'une quelconque des revendications 1 à 7.

9. Cellule solaire sensibilisée avec un colorant suivant la revendication 8, dans laquelle la couche absorbant la lumière comprend en outre au moins un additif représenté par la formule 4 :
Z-COOH (4)
dans laquelle Z représente une des entités consistant en un atome d'hydrogène, un groupe hydroxy, un atome d'halogène, un groupe nitro, un groupe cyano, un groupe carboxyle, un groupe amino substitué ou non substitué, un groupe acyle substitué ou non substitué, un groupe acyloxy substitué ou non substitué, un groupe alkyle substitué ou non substitué, un groupe cycloalkyle substitué ou non substitué, un groupe halogénalkyle substitué ou non substitué, un groupe alkylsulfonyle substitué ou non substitué, un groupe arylsulfonyle substitué ou non substitué, un groupe alkylthio substitué ou non substitué, un groupe alkoxy substitué ou non substitué, un groupe alkoxysulfonyle substitué ou non substitué, un groupe alkoxycarbonyle substitué ou non substitué, un groupe aryle substitué ou non substitué, un groupe aryloxy substitué ou non substitué, un groupe alcényle substitué ou non substitué, un groupe arylalkyle substitué ou non substitué ou un groupe hétérocyclique substitué ou non substitué.

10. Cellule solaire sensibilisée avec un colorant suivant la revendication 9, dans laquelle l'additif est un des composés consistant en l'acide désoxycholique, l'acide phénylpropionique, l'acide dodécylmalonique, et l'acide dodécylphosphonique.

11. Cellule solaire sensibilisée avec un colorant suivant la revendication 9 ou 10, dans laquelle l'additif est inclus en une quantité de 100 à 3000 parties en poids, sur la base de 100 parties en poids du colorant pour une cellule solaire sensibilisée avec un colorant.

12. Cellule solaire sensibilisée avec un colorant suivant l'une quelconque des revendications 8 à 11, dans laquelle la couche absorbant la lumière a une épaisseur égale ou inférieure à 25 µm.
